# EUROPÄISCHE PATENTSCHRIFT

(11) **EP 2 630 117 B1**
(45) Veröffentlichungstag und Bekanntmachung des Hinweises auf die Patenterteilung: **15.10.2014**
(21) Anmeldenummer: 11773236.2
(22) Anmeldetag: 17.10.2011
(51) Int. Cl.: C07C 209/84, C07C 211/46

(54) **VERFAHREN ZUR REINIGUNG VON ANILIN AUS GASPHASENHYDRIERUNGEN**
PROCESS FOR PURIFYING ANILINE FROM GAS PHASE HYDROGENATIONS
PROCÉDÉ DE PURIFICATION D'ANILINE PROVENANT D'HYDROGÉNATIONS EN PHASE GAZEUSE

(30) Priorität: 21.10.2010 DE 102010042731
(43) Veröffentlichungstag der Anmeldung: 28.08.2013
(73) Patentinhaber: Bayer Intellectual Property GmbH, 40789 Monheim am Rhein (DE)
(72) Erfinder: LEHNER, Peter, 77523-0000 Baytown Texas (US); SOMMER, Knut, 47804 Krefeld (DE); MOUSSA, Amgad Salah, 51061 Köln (DE)
(74) Vertreter: BIP Patents
(86) Internationale Anmeldenummer: PCT/EP2011/068122
(87) Internationale Veröffentlichungsnummer: WO 2012/052407

(56) Entgegenhaltungen:
- EP-A1- 1 845 080

## Beschreibung

Die Erfindung betrifft ein Verfahren zur Reinigung von durch Gasphasenhydrierung von Nitrobenzol hergestelltem Anilin durch fraktionierende Kondensation des gasförmig anfallenden rohen Reaktionsproduktes, sodass mindestens zwei flüssige Verfahrensprodukte, ein *Partialkondensat* (PK) und ein *Totalkondensat* (TK) erhalten werden, wobei die zur Bildung von PK führende Kondensation bei höherer Kondensation durchgeführt wird als die zur Bildung von TK führende Temperatur, ein aus PK und ein aus TK stammender Produktstrom jeweils getrennt voneinander in eine aus mindestens einem Abtriebsteil und mindestens einem Verstärkungsteil bestehende Destillationskolonne geleitet werden, wobei der aus PK stammende Produktstrom in den unteren Teil der Destillationskolonne zwischen dem untersten Abtriebsteil und der darauf folgenden Sektion und der aus TK stammende Produktstrom in den Kopf der Destillationskolonne oberhalb des obersten Verstärkungsteils eingeleitet werden, und das gewünschte gereinigte Anilin in einem Seitenstrom zwischen unterstem Abtriebsteil und obersten Verstärkungsteil der Destillationskolonne entnommen wird.

Aromatische Amine sind wichtige Zwischenprodukte, welche preiswert und in großen Mengen zur Verfügung stehen müssen. Anilin, ein technisch besonders bedeutsames aromatisches Amin, kann hervorragend nach dem erfindungsgemäßen Verfahren gereinigt werden. Anilin ist ein wichtiges Zwischenprodukt bei der Herstellung von Di- und Polyisocyanaten der Diphenylmethanreihe (MDI) und wird im großtechnischen Maßstab in der Regel durch katalytische Hydrierung von Nitrobenzol hergestellt. Hierfür müssen Anlagen mit sehr großen Kapazitäten gebaut werden, um den enormen weltweiten Bedarf decken zu können. Bevorzugt wird die Hydrierung von Nitrobenzol in der Gasphase an ortsfesten, heterogenen Trägerkatalysatoren, wie z. B. Pd auf Aluminiumoxid oder Kohleträgern, in Festbettreaktoren bei einem absoluten Druck von 2 - 50 bar und einer Temperatur im Bereich von 250 - 500 °C unter adiabatischen Bedingungen in Kreisgasfahrweise durchgeführt; siehe EP-A-0 696 573, EP-A-0 696 574 und EP-A-1 882 681. "Kreisgasfahrweise" bedeutet, dass die im rohen Reaktionsprodukt enthaltenen nichtkondensierbaren Gase (also im Wesentlichen während der Hydrierung nicht umgesetzter Wasserstoff und ggf. zugesetzte Inertgase) ggf. mit Ausnahme geringer, zum Konstanthalten von weiteren gasförmigen Komponenten des Kreisgases - wie etwa von auf dem Katalysator durch Desaminierungsreaktionen gebildetes Ammoniak - abgezweigter Mengen, in die Reaktion zurückgeführt werden.

Bei der Herstellung von Anilin werden neben dem Zielprodukt auch Wasser und organische Nebenkomponenten gebildet. Darüber hinaus können je nach Produktionsverfahren auch noch Anteile an nicht umgesetztem Nitrobenzol enthalten sein. Diese organischen Nebenkomponenten sowie ggf. nichtumgesetztes Nitrobenzol müssen vor einer weiteren Verwendung des Anilins abgetrennt werden. Die organischen Nebenkomponenten sowie ggf. nichtumgesetztes Nitrobenzol können in zwei Gruppen unterteilt werden: a) die Gruppe der "Leichtsieder", d. h. Verbindungen bzw. azeotrop siedende Gemische von Einzelkomponenten mit Siedepunkten unter denen von Anilin (Sdp. = 184 °C), und b) die Gruppe der "Hochsieder", d. h. Verbindungen bzw. azeotrop siedende Gemische von Einzelkomponenten mit Siedepunkten über denen von Anilin. Nitrobenzol (Sdp. = 211 °C) gehört demnach zur Gruppe der Hochsieder.

Ein roher Produktstrom einer Gasphasenhydrierungsanlage von Nitrobenzol besteht also in der Regel aus
- Anilin,
- Prozesswasser (d. i. die Summe aus in der Reaktion gebildetem und ggf. dem Eduktgasstrom zugesetztem Wasser),
- nichtkondensierbaren Gasen (Überschusswasserstoff - ggf. enthaltend gasförmige Verunreinigungen wie bspw. Methan, Kohlenstoffoxide - und ggf. zugesetzte Inertgase, z. B. zur Selektivitätsverbesserung zugesetzter Stickstoff, vgl. EP-A-1 882 681, und ggf. gasförmige Nebenprodukte, z. B. Ammoniak aus Desaminierungsreaktionen),
- Leichtsiedern und
- Hochsiedern (die ggf. auch Anteile an nichtumgesetztem Nitrobenzol enthalten können).

Beispiele für die Gruppe der Leichtsieder sind das Benzol (Sdp. = 80 °C) und für die Gruppe der Hochsieder das Diphenylamin (Sdp. = 302 °C). Von diesen beiden als Beispiele genannten Verunreinigungen lässt sich das Anilin einfach abtrennen, weil ihre Siedepunkte von dem des Anilins sehr verschieden sind (ΔTₛ = 104 K bzw. 118 K). Andererseits sind es gerade die Hochsieder, welche nach der Kondensation des Produkts eine erneute Verdampfung und erneute Kondensation des Anilins selbst überhaupt erst erforderlich machen, sodass deren Vorhandensein in besonderer Weise problematisch ist.

Eine besondere Schwierigkeit stellt darüber hinaus die Abtrennung von solchen Nebenkomponenten dar, deren Siedepunkte denen des Anilins sehr ähnlich sind, weil hier der destillative Aufwand erheblich ist. In diesem Zusammenhang stellt insbesondere die Abtrennung von Phenol (Sdp. = 182 °C) eine große Herausforderung für die Destillationstechnik dar, was sich in der Verwendung langer Destillationskolonnen mit großer Trennstufenzahl und hohen Rücklaufverhältnissen mit entsprechend hohem Investitions- und Energieaufwand widerspiegelt. Verbindungen mit phenolischen Hydroxygruppen, d. h. Verbindungen, die mindestens eine Hydroxygruppe (-OH) direkt an einem aromatischen Ring tragen, können generell bei der Aufarbeitung von Anilin problematisch sein. Neben dem bereits erwähnten Phenol sind insbesondere die verschiedenen Aminophenole zu nennen.

Die Reinigung von Anilin ist daher nicht trivial und besitzt große industrielle Bedeutung. Viele Ansätze widmen sich insbesondere der erwähnten Problematik im Zusammenhang mit Verbindungen mit phenolischen Hydroxygruppen. Der Lösungsansatz besteht darin, die Verbindungen mit phenolischen Hydroxygruppen durch Reaktion mit geeigneten Basen in die entsprechenden Salze zu überführen, welche sich als nichtflüchtige Verbindungen wesentlich leichter abtrennen lassen.

So offenbaren JP-A-49-035341**,** US-A-2005 080294**,** EP-A-1 845 079 und EP-A-2 028 176 Verfahren, bei denen ein aromatisches Amin *in Gegenwart einer Base* destilliert wird. Bei dieser Verfahrensweise müssen Probleme durch Feststoffabscheidung, Fouling und/oder starken Viskositätsanstieg bei der Destillation durch aufwändige und/oder teure Maßnahmen verhindert werden.

Als Alternative zur Entfernung von Verbindungen mit phenolischen Hydroxygruppen aus Anilin während der Destillation beschreibt JP-A-08-295654 eine Extraktion mit verdünnter wässriger Alkalihydroxidlösung. Nachteilig bei diesem Verfahren sind der hohe NaOH-Verbrauch und das Anfallen - infolge der geringen Konzentration der Alkalihydroxid-Lösungen - sehr großer Mengen alkaliphenolathaltigen Abwassers.

EP-A-1 845 080 beschreibt ein Verfahren zur Reinigung von Anilin durch Extraktion mit wässriger Alkalimetallhydroxid-Lösung einer Konzentration > 0,7 Massen-%, wobei Konzentration und Temperatur so eingestellt werden, dass die wässrige Phase bei der anschließenden Phasentrennung immer die untere Phase darstellt.

JP-A-2007217405 beschreibt ein Verfahren, in dem das phenolhaltige Anilin mindestens zweimal mit wässriger Alkalimetallhydroxidlösung so in Kontakt gebracht wird, dass die Konzentration an Alkalimetallhydroxid in der wässrigen Phase zwischen 0,1 Massen-% und 0,7 Massen-% liegt.

Ganz allgemein mit der Verbesserung der Anilinaufarbeitung befasst sich JP-A-2005 350388. Es wird ein Verfahren beschrieben, bei dem ein Teil des Sumpfes der Anilin-Destillationskolonne aus dieser abgeführt und separat, d. h. in einem vom eigentlichen Verdampfer der Kolonne verschiedenen zweiten Verdampfer, in die Gasphase überführt wird. Die so erhaltene Gasphase wird in die Reinanilinkolonne zurückgeführt; nicht verdampfbare Hochsiederanteile werden abgetrennt. Nachteilig bei diesem Verfahren ist, dass Leichtsieder und Wasser vor der eigentlichen Anilin-Destillationskolonne in einem apparativ aufwändigen Verfahren in einer Entwässerungskolonne separat durch eine zusätzliche Destillation abgetrennt werden müssen.

Keine der genannten Druckschriften geht darauf ein, wie es erreicht werden kann, den Anteil des Anilins, der in einem Destillationsprozess verdampft und erneut kondensiert werden muss, zu verringern. Stammt das zu reinigende Anilin aus einem Gasphasenprozess, so durchläuft es nach dem Stand der Technik sogar zwei Kondensationen: Zunächst wird das gasförmig anfallende Reaktionsprodukt möglichst vollständig kondensiert, die wässrige Phase abgetrennt und die erhaltene organische Phase destilliert, d. h. das gewünschte Produkt wird (i) kondensiert, (ii) verdampft und (iii) wieder kondensiert, was energetisch und apparativ sehr aufwändig ist und zu erheblichen thermischen Belastungen des Anilins führt.

Eine Aufgabe der vorliegenden Erfindung war es daher, ein Verfahren zur Reinigung von aus Gasphasenhydrierungen stammendem Anilin bereitzustellen, bei dem nur ein möglichst geringer Anteil des Anilins selbst erneut wieder verdampft und kondensiert werden muss und bei dem die Abtrennung von Verbindungen mit phenolischen Hydroxygruppen möglichst effektiv mit möglichst geringen Verlusten an wertvollem Anilin erreicht wird.

Die Aufgabe wurde gelöst durch ein Verfahren zur Herstellung von Anilin durch Gasphasenhydrierung von Nitrobenzol und anschließende Reinigung des Anilins, worin ein gasförmiges rohes Reaktionsprodukt, bestehend aus Anilin, Prozesswasser, nichtkondensierbaren Gasen, Leichtsiedern und Hochsiedern, anfällt, welches fraktionierend kondensiert wird, sodass mindestens zwei, bevorzugt genau zwei flüssige Verfahrensprodukte, nämlich wenigstens ein, bevorzugt ein *Partialkondensat* (PK) und ein *Totalkondensat* (TK) erhalten werden, wobei
die zur Bildung von PK führende Kondensation bei höherer Temperatur durchgeführt wird als die zur Bildung von TK führende Kondensation, PK den Hauptanteil der Hochsieder enthält, während TK den Hauptanteil der Leichtsieder sowie den Hauptanteil des Anilins und den Hauptanteil des Prozesswassers enthält, dadurch gekennzeichnet, dass
ein aus PK stammender Produktstrom und ein aus TK stammender Produktstrom in eine Destillationskolonne umfassend mindestens zwei Sektionen, nämlich mindestens ein Abtriebsteil (**AT**) und mindestens ein Verstärkungsteil (**VT**), geleitet werden, wobei
der aus PK stammende Produktstrom in den unteren Teil der Destillationskolonne zwischen dem untersten Abtriebsteil und der darauf folgenden Sektion und der aus TK stammende Produktstrom in den Kopf der Destillationskolonne oberhalb des obersten Verstärkungsteils eingeleitet werden, und wobei
destilliertes Anilin in einem Seitenstrom zwischen unterstem Abtriebsteil und oberstem Verstärkungsteil der Destillationskolonne entnommen wird.

Das Verfahren wird im Folgenden anhand der bevorzugten Ausführungsform, welche die Bildung von genau zwei Kondensatströmen (PK und TK) zum Gegenstand hat, erläutert. Es ist für den Fachmann ein Leichtes, dieses Verfahren so zu modifizieren, dass es mehr als zwei Kondensatströme umfasst, etwa indem der möglichst vollständigen letzten Kondensationsstufe (TK) mehrere partielle Kondensationsstufen PK₁, PK₂ usw. vorgeschaltet sind.

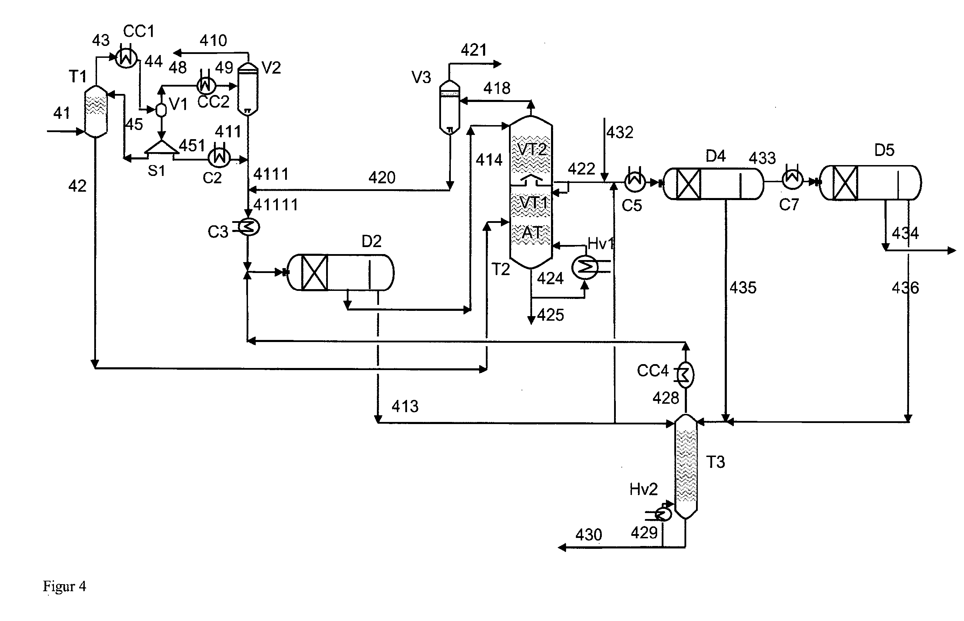

In der einfachsten Ausführungsform des erfindungsgemäßen Verfahrens ist der aus PK stammende Produktstrom das in der fraktionierenden Kondensation erhaltene Partialkondensat, d. h. er ist identisch mit PK selbst und der aus TK stammende Produktstrom ist die organische Phase, die durch Phasentrennung von TK oder eines Verfahrensproduktes enthaltend TK, gewonnen durch Vereinigung von TK mit weiteren Produktströmen (siehe Figur 4 und die zugehörigen Erläuterungen), in eine wässrige und eine organische Phase erhalten wird.

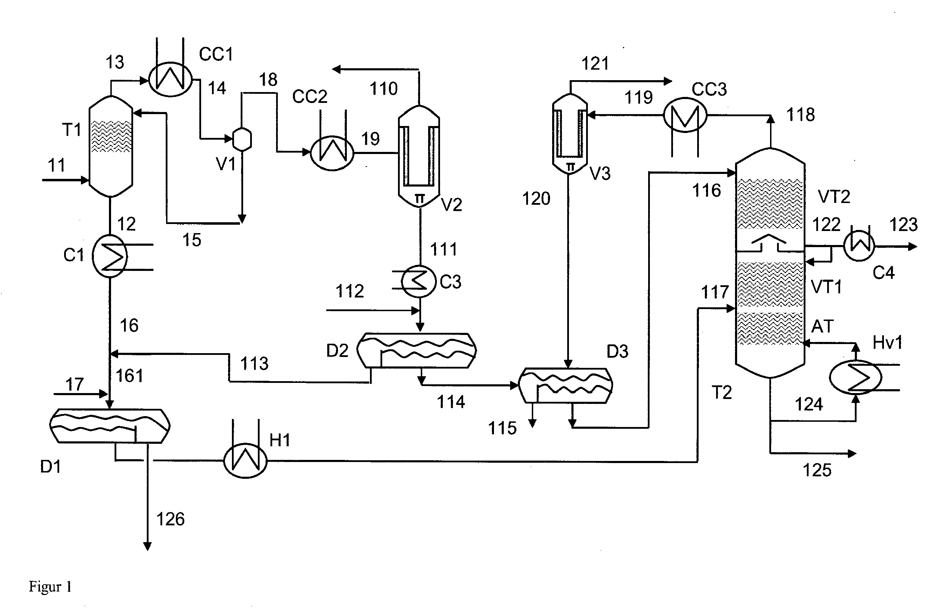

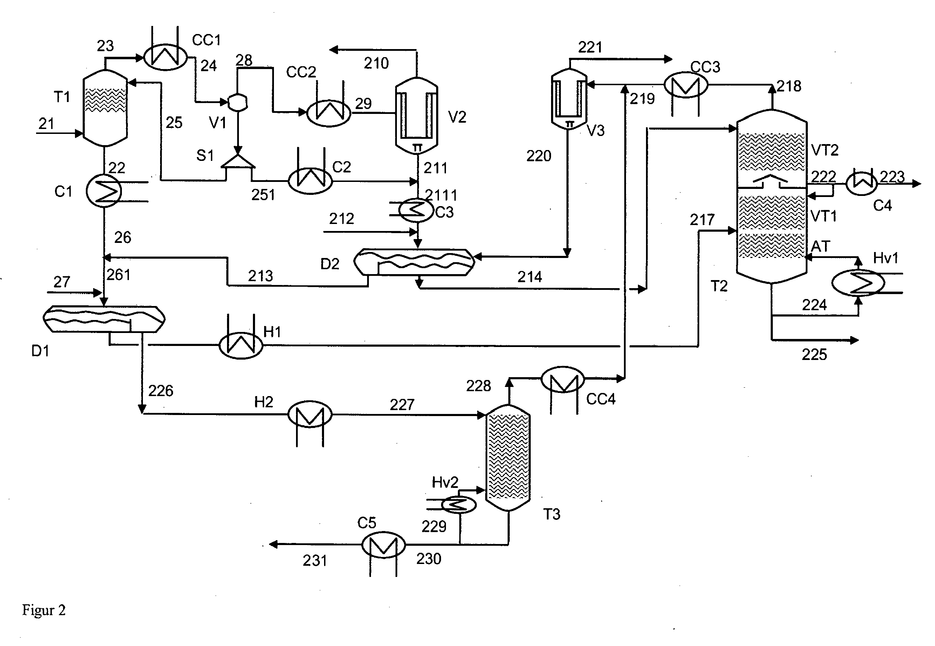

Abhängig von der genauen Trennaufgabe (d. h. letztlich vom Nebenproduktspektrum, welches je nach Herstellverfahren variiert) kann es vorteilhaft sein, das Anilin mit Base zu behandeln, um saure Verunreinigungen, insbesondere die erwähnten Verbindungen mit phenolischen Hydroxygruppen, noch vollständiger zu entfernen, als es ansonsten möglich wäre. In einem solchen Fall ist der aus PK stammende Produktstrom bevorzugt eine organische Phase, die nach Vermischung von PK oder eines Verfahrensproduktes enthaltend PK, gewonnen durch Vereinigung von PK mit weiteren Produktströmen (siehe Figuren 1 bis 3 und die zugehörigen Erläuterungen), mit wässriger Baselösung und anschließender Phasentrennung in eine wässrige und eine organische Phase erhalten wird. Analog ist in einem solchen Fall der aus TK stammende Produktstrom bevorzugt eine organische Phase, die nach Vermischung von TK oder eines Verfahrensproduktes enthaltend TK (siehe Figuren 2 bis 3 und die zugehörigen Erläuterungen) mit wässriger Baselösung und anschließender Phasentrennung in eine wässrige und eine organische Phase erhalten wird.

Besonders bevorzugt wird in dieser Ausführungsform PK oder das Verfahrensprodukt enthaltend PK *vor, während* oder *nach* der Vermischung mit wässriger Baselösung zusätzlich in einem ein-oder mehrstufigen Prozess mit Wasser oder einem mindestens 50 Massen-% Wasser, bevorzugt mindestens 80 Massen-% Wasser, besonders bevorzugt mindestens 90 Massen-% Wasser enthaltenden Strom A, bezogen auf die Gesamtmasse dieses Stromes A, vermischt, um die Beladung des aus PK stammenden Produktstroms mit Salzen soweit wie möglich zu reduzieren. Ganz besonders bevorzugt ist dieser Strom A eine wässrige Phase, die nach Vermischung von TK oder eines Verfahrensproduktes enthaltend TK mit wässriger Baselösung und anschließender Phasentrennung in eine wässrige und eine organische Phase erhalten wird.

Ebenfall bevorzugt ist es, TK oder das Verfahrensprodukt enthaltend TK *vor*, *während* oder *nach* der Vermischung mit wässriger Baselösung (jedoch noch vor der Phasentrennung) zusätzlich in einem ein- oder mehrstufigen Prozess mit Wasser oder einem mindestens 50 Massen-% Wasser, bevorzugt mindestens 80 Massen-% Wasser, besonders bevorzugt mindestens 90 Massen-% Wasser enthaltenden Strom B, bezogen auf die Gesamtmasse dieses Stromes B, zu vermischen. Alternativ ist es auch möglich, die nach der Vermischung von TK oder eines Verfahrensproduktes enthaltend TK mit wässriger Baselösung *und anschließender Phasentrennung* erhaltene organische Phase in einem ein- oder mehrstufigen Prozess mit Wasser oder einem mindestens 50 Massen-% Wasser, bevorzugt mindestens 80 Massen-% Wasser, besonders bevorzugt mindestens 90 Massen-% Wasser enthaltenden Strom B zu waschen. In beiden Fällen wird als Strom B bevorzugt ein flüssiger Kondensatstrom der über Kopf der Destillationskolonne abgezogenen Gasphase oder ein einen flüssigen Kondensatstrom der über Kopf der Destillationskolonne abgezogenen Gasphase enthaltendes Verfahrensprodukt verwendet. Ein "einen flüssigen Kondensatstrom der über Kopf der Destillationskolonne abgezogenen Gasphase enthaltendes Verfahrensprodukt" ist ganz besonders bevorzugt ein solches, welches durch Zugabe eines Anilin-Wasser-Azeotrops (seinerseits erhalten durch Einleitung einer durch Phasentrennung nach der Vermischung von PK oder eines Verfahrensproduktes enthaltend PK mit wässriger Baselösung erhaltenen wässrigen Phase in einen Wasserstripper) zum Gesamtkondensat der über Kopf der Destillationskolonne abgezogenen Gasphase erhalten wird.

Alternativ kann die Behandlung des Anilins mit wässriger Baselösung auch nach der Destillation erfolgen. In dieser Ausführungsform ist bevorzugt der aus PK stammende Produktstrom identisch mit PK selbst, und der aus TK stammende Produktstrom ist bevorzugt die organische Phase, die durch Phasentrennung eines Verfahrensproduktes enthaltend TK (siehe Figur 4 und die zugehörigen Erläuterungen) in eine wässrige und eine organische Phase erhalten wird. Das der Destillationskolonne in einem Seitenstrom entnommene Anilin wird mit wässriger Baselösung vermischt, und ein an sauren Verunreinigungen abgereichertes Anilin wird durch eine Phasentrennung gewonnen.

Eine erfindungsgemäße Destillationskolonne umfasst mindestens zwei Sektionen, einen Abtriebsteil und einen Verstärkungsteil. Im einfachsten Fall wird daher der aus PK stammende Produktstrom zwischen diese beiden Sektionen eingeleitet, und der aus TK stammende Produktstrom wird in den Kopf der Kolonne oberhalb des Verstärkungsteils geleitet. Umfasst die Destillationskolonne mehr als zwei Sektionen, so wird der aus PK stammende Produktstrom bevorzugt zwischen dem untersten Abtriebsteil und die nächst folgende Sektion, im Allgemeinen ein Verstärkungsteil, geleitet. Bei drei Sektionen (siehe Figuren), **AT** (= unterster, in dieser Ausführungsform einziger Abtriebsteil), **VT1** (= auf **AT** folgender Verstärkungsteil) und **VT2** (= oberster Verstärkungsteil) wird der aus TK stammende Produktstrom zwischen **AT** und **VT1** eingeleitet, und der aus **TK** stammende Produktstrom wird in den Kopf der Kolonne oberhalb von **VT2** eingeleitet. Die Zahl der Sektionen kann auch höher als drei sein.

Eine mögliche Ausführungsform des erfindungsgemäßen Verfahrens wird im Folgenden mit Hilfe von Figur 1 näher erläutert.

### Es bedeuten:

(Die erste Ziffer eines Bezugszeichens eines Stoffstromes zeigt in dieser Schrift die zugehörige Figur an. Bei identischen oder vergleichbaren Stoffströmen aus unterschiedlichen Ausführungsformen der Erfindung sind die der ersten Ziffer folgenden Ziffern des jeweiligen Bezugszeichens gleich.)

**Tabelle 1: Bezugszeichen der Figur 1.**

| **Stoffstrom** | **Bedeutung** | **Apparat** | **Bedeutung** |
|---|---|---|---|
| **11** | Gasförmiges rohes Reaktionsprodukt (Rohanilin) | **T1** | Wäscher zur Aufteilung des Rohanilinstroms in PK und TK |
| **12** | Aus **T1** ausgetragenes flüssiges Verfahrensprodukt (PK) | **CC1** | Kondensator |
| **13** | Kopfprodukt aus **T1** | **V1** | Abscheider |
| **14** | **13** nach Durchlaufen von **CC1** | **CC2** | Kondensator |
| **15** | In den Kopf von **T1** zurückgeführter flüssiger Produktstrom | **V2** | Abscheider |
| **16** | **12** nach Durchlaufen von **C1** | **C3** | Kühler |
| **161** | **16** nach Vermischung mit **113** (161 = *"Verfahrensprodukt enthaltend PK"*) | **D2** | Dekanter |
| **17** | Wässrige Baselösung zur Behandlung von PK oder eines Verfahrensproduktes enthaltend PK | **C1** | Kühler |
| **18** | Kopfprodukt aus V1 | **D1** | Dekanter |
| **19** | **18** nach Durchlaufen von **CC2** | **H1** | Vorwärmer |
| **110** | Kopfprodukt aus **V2** | **D3** | Dekanter |
| **111** | Aus **V2** ausgetragenes flüssiges Verfahrensprodukt (TK) | **T2** | Destillationskolonne |
| **112** | Wässrige Baselösung zur Behandlung von TK oder eines Verfahrensproduktes enthaltend TK | **AT** | Abtriebsteil |
| **113** | Wässrige Phase aus **D2** | **VT1** | Oberes Verstärkungsteil |
| **114** | Organische Phase aus **D2** | **VT2** | Mittleres Verstärkungsteil |
| **115** | Wässrige Phase aus **D3** | **Hv1** | Verdampfer |
| **116** | Organische Phase aus **D3** | **C4** | Kühler |
| **117** | Organische Phase aus **D1** | **CC3** | Kondensator |
| **118** | Aus **T2** über Kopf abgezogene Gasphase | **V3** | Abscheider |
| **119** | **118** nach Durchlaufen von **CC2** (Gesamtkondensat von 118) | | |
| **120** | Aus **V3** unten abgezogener flüssiger Kondensatstrom | | |
| **121** | Kopfprodukt aus **V3** | | |
| **122** | Anilinstrom aus Seitenentnahme von **T2** | | |
| **123** | Anilinstrom aus Seitenentnahme von **T2** nach Durchlaufen von **C4** | | |
| **124** | Umlaufstrom zum Verdampfer **Hv1** | | |
| **125** | Ausgeschleuster Bodenablauf aus **T2** | | |
| **126** | Wässrige Phase aus **D1** | | |

Das gasförmige Reaktionsprodukt, Strom **11**, bestehend Anilin, Prozesswasser, nichtkondensierbaren Gasen, Leichtsiedern und Hochsiedern wird zunächst in den Wäscher **T1** geleitet. Am Kopf von **T1** wird Strom **13** entnommen und im Kondensator **CC1** auf eine Temperatur von 60 °C bis 160 °C, bevorzugt von 80 °C bis 140 °C und besonders bevorzugt von 100 °C bis 120 °C abgekühlt *(zur Bildung von PK führende Kondensation).* Der absolute Druck liegt dabei zwischen 1 bar und 50 bar, bevorzugt zwischen 2 bar und 20 bar und besonders bevorzugt zwischen 2 bar und 10 bar.

Der so erhaltene Strom **14** wird im Abscheider **V1** in eine gasförmige und eine flüssige Phase getrennt. Der in den Kopf von **T1** zurückgeleitete Strom **15** ist in dieser Ausführungsform die unten aus **V1** abgezogene Flüssigphase. In **T1** bildet sich auf diese Weise ein an Hochsiedern angereichertes flüssiges Verfahrensprodukt (PK), welches unten abgezogen wird (Strom **12**). Strom **12** (PK) enthält neben den Hochsiedern (bevorzugt 90 Massen-% bis 100 Massen-% der in Strom **11** enthaltenen Hochsieder) noch Anteile an Anilin (bevorzugt 0,1 Massen-% bis 35 Massen-% des in Strom **11** enthaltenen Anilins), an Leichtsiedern (bevorzugt <1 Massen-% der in Strom **11** enthaltenen Leichtsieder) sowie an Prozesswasser (bevorzugt < 5 Massen-% des in Strom **11** enthaltenen Prozesswassers). Dieser (Strom **12** wird, bevorzugt nach Durchlaufen eines Kühlers **C1** (dann Strom **16**), in einem ein- oder mehrstufigen Prozess mit wässriger Baselösung **17** und Wasser (nicht gezeigt) oder einem mindestens 50 Massen-%, bevorzugt mindestens 80 Massen-%, besonders bevorzugt mindestens 90 Massen-% Wasser enthaltenden Strom A (bevorzugt Strom **113**) vermischt und dem Dekanter **D1** zugeführt. Das Vermischen mit Wasser oder einem mindestens 50 Massen-% Wasser enthaltenden Strom kann vor, gleichzeitig oder nach dem Vermischen mit Baselösung geschehen, bevorzugt danach.

Grundsätzlich sind alle wasserlöslichen basischen Verbindungen zur Herstellung der erfindungsgemäßen wässrigen Baselösungen geeignet, wie z. B. Alkalimetall- oder Erdalkalimetallhydroxide, -carbonate oder -hydrogencarbonate. Bevorzugt wird als Baselösung eine Lösung von Natriumhydroxid in Wasser eingesetzt, wobei der Massenanteil an Natriumhydroxid bevorzugt mindestens 10 %, besonders bevorzugt mindestens 20 % und ganz besonders bevorzugt mindestens 30 %, bezogen auf die Gesamtmasse der Natriumhydroxidlösung, beträgt. Außerordentlich besonders bevorzugt wird kommerziell erhältliche 32%ige Natronlauge eingesetzt. Das molare Verhältnis von Base zur Summe aller phenolischen Hydroxygruppen beträgt mindestens 1:1, bevorzugt wird jedoch ein molarer Überschuss an Base eingesetzt. Dabei wird ein molarer Überschuss von Base zur Summe aller phenolischen Hydroxygruppen von maximal 20 : 1, bevorzugt von maximal 15 : 1 und besonders bevorzugt von maximal 10 : 1 eingehalten. Bei mehrstufigen Basenbehandlungen wird dieses Molverhältnis bevorzugt in jeder Stufe eingehalten. Um das gewünschte molare Verhältnis von Base zur Summe aller phenolischen Hydroxygruppen einstellen zu können, muss die Zusammensetzung des zu behandelnden Verfahrensstromes bekannt sein. Diese wird durch gängige analytische Methoden ermittelt, bevorzugt durch Gaschromatographie. Die vorstehend gemachten Angaben zu Art und Menge der einzusetzenden Base gelten für alle Ausführungsformen des erfindungsgemäßen Verfahrens. Das durch die Basebehandlung und Waschen mit Wasser erhaltene zweiphasige Verfahrensprodukt wird im Dekanter **D1** einer dem Fachmann bekannten Phasentrennung unterworfen, wobei Salze (überschüssige Base und Salze der Verbindungen mit phenolischen Hydroxygruppen) überwiegend bis vollständig in der wässrigen Phase verbleiben. Diese wässrige Phase (Strom **126**) wird bevorzugt einer Abwasserbehandlung zugeführt, besonders bevorzugt einem Abwasserstripper.

Der aus dem Kopf von **V1** abgezogene gasförmige Strom **18** wird im Kondensator **CC2** kondensiert, sodass sich ein flüssiges Verfahrensprodukt (*zur Bindung von TK führende Kondensation*) neben einer verbleibenden Gasphase bildet. Die Kondensationstemperatur richtet sich nach den ökonomischen Randbedingungen: Eine zu hohe Temperatur führt zu unerwünschten Produktverlusten, eine zu geringe Temperatur zu unvertretbarem Energieaufwand für die Kondensation. Die tatsächlich gewählte Kondensationstemperatur stellt daher einen Kompromiss dar und liegt bevorzugt zwischen 25 °C und 90 °C, besonders bevorzugt zwischen 30 °C und 80 °C und ganz besonders bevorzugt zwischen 40 °C und 70 °C. In diesem Verfahrensschritt kondensiert nicht nur Anilin, sondern es kondensieren auch Wasser und Leichtsieder, sowie ggf. geringe Anteile an mitgeschleppten Hochsiedern, aus. Der absolute Druck liegt dabei zwischen 1 bar und 50 bar, bevorzugt zwischen 2 bar und 20 bar und besonders bevorzugt zwischen 2 bar und 10 bar.

Der aus **CC2** austretende Strom **19** wird in den Abscheider **V2** geleitet, um die flüssige Phase und gasförmige Phase zu trennen. Die gasförmige Phase, die den aus der Reaktion stammenden Überschusswasserstoff enthält, wird bevorzugt als Kreisgas in die Nitroaromatenreduktion zurückgeführt (Strom **110**). Das als Strom **111** abgezogene flüssige Verfahrensprodukt (TK) wird, bevorzugt nach Durchlaufen eines weiteren Kühlers **C3**, in einem ein- oder mehrstufigen Prozess mit Baselösung (Strom **112**) vermischt, wobei bevorzugt die gleiche Art Baselösung wie in Strom **17** eingesetzt wird und der molare Überschuss an Base bevorzugt der gleiche ist wie bei der Basebehandlung von Strom **12** bzw. (in der bevorzugten Ausführungsform) Strom **16**. Die analytische Untersuchung des mit Base zu behandelnden Stromes erfolgt bevorzugt mit der gleichen Methode wie im Fall von Strom **12** bzw. (in der bevorzugten Ausführungsform) Strom **16**. Neben der Hauptmenge des Anilins (bevorzugt 65 Massen-% bis 99,9 Massen-% des in Strom **11** enthaltenen Anilins), der Hauptmenge der Leichtsieder (bevorzugt 99 Massen-% bis 100 Massen-% der in Strom **11** enthaltenen Leichtsieder) und der Hauptrnenge des Prozesswassers (bevorzugt 95 Massen-% bis 100 Massen-% des in Strom **11** enthaltenen Prozesswassers) können in Strom **111** (TK) noch geringe Anteile an mitgeschleppten Hochsiedern (bevorzugt < 10 Massen-% der in Strom **11** enthaltenen Hochsieder) enthalten sein. Das zweiphasige Verfahrensprodukt wird nun im Dekanter **D2** einer dem Fachmann bekannten Phasentrennung unterworfen. Die dabei erhaltene wässrige Phase enthält die Salze der Verbindungen mit phenolischen Hydroxygruppen sowie überschüssige Base teilweise bis vollständig. Diese wässrige Phase wird aus **D2** abgeführt (Strom **113**). In einer besonders bevorzugten Ausführungsform des erfindungsgemäßen Verfahrens wird bevorzugt dieser Strom **113** als mindestens 50 Massen-% Wasser enthaltender Strom A zum Waschen des Stromes **16** vor, während oder nach der Basebehandlung von Strom **16** verwendet.

Die alkalische Behandlung im Dekanter **D2** kann als ein- oder mehrstufige Behandlung mit wässriger Baselösung **112** ausgeführt werden. Die durch die Phasentrennung in **D2** erhaltene organische Phase wird aus **D2** abgeführt (Strom **114**) und bevorzugt, um auch die letzten Reste an Salzen zu entfernen, in einem ein- oder mehrstufigen Prozess mit Wasser (in Figur 1 nicht gezeigt) oder einem mindestens 50 Massen-% Wasser, bevorzugt mindestens 80 Massen-% Wasser, besonders bevorzugt mindestens 90 Massen-% Wasser enthaltenden Strom B (= **120**) gewaschen. Das auf diese Weise erhaltene zweiphasige Verfahrensprodukt wird im Dekanter **D3** einer dem Fachmann bekannten Phasentrennung unterworfen. Die Wäsche im Dekanter **D3** kann als ein- oder mehrstufige Wäsche ausgeführt werden. Die wässrige Phase aus der Wäsche (Strom **115**) wird in dieser Ausführungsfonn ausgeschleust und kann anschließend bevorzugt einer weiteren Prozessstufe (nicht gezeigt) zugeführt werden, in der Strom **115** weiter aufgereinigt und das in diesem Strom enthaltene Anilin zurückgewonnen wird Verbleibende wässrige Anteile werden einer Abwasserentsorgung zugeführt.

Die Destillationskolonne **T2** besteht bevorzugt aus einem oberen Verstärkungsteil, einem mittleren Verstärkungsteil und einem unteren Abtriebsteil sowie einem Verdampfer **Hv1**.

Der aus PK stammende Produktstrom, der in den unteren Teil der Destillationskolonne **T2** zwischen dem untersten Abtriebsteil und der darauf folgenden Sektion eingeleitet wird, ist in dieser Ausführungsform die in **D1** erhaltene organische Phase. Diese wird bevorzugt im Vorwärmer **H1** auf 100 °C bis 180 °C erhitzt und dann in **T2** eingeleitet (Strom **117**).

Der aus TK stammende Produktstrom, der in den Kopf der Destillationskolonne **T2** oberhalb des obersten Verstärkungsteils eingeleitet wird, ist in dieser Ausführungsform die in **D3** erhaltene organische Phase (Strom **116**). Strom **116** hat bevorzugt eine Temperatur von 30 °C bis 60 °C-Das Anilin wird der Destillationskolonne als Seitenstrom (**122**) entnommen. Ein Teil dieses Anilins (bevorzugt 10 Massen-% bis 80 Massen-%, besonders bevorzugt 20 Massen-% bis 50 Massen-% des Stroms **122**) wird bevorzugt in die Destillationskolonne zurückgeführt, um den Gasstrom in derselben auf einem Niveau zu halten, das es erlaubt, die Leichtsieder ausreichend abzutrennen. Bevorzugt wird der nicht zurückgeführte Anteil des Anilins im Kühler **C4** weiter abgekühlt und als Reinanilinstrom **123** entnommen.

Der über Kopf entnommene Strom **18** enthält neben Anteilen an Anilin Leichtsieder und Restwasser, da Wasser aufgrund einer gewissen Löslichkeit in der organischen Phase in den Dekantern nicht vollständig abgetrennt werden kann. Nach Durchlaufen des Kondensators **CC3** wird Strom **119** in **V3** eingespeist, wo unten ein wasserreiches Kondensat (Strom **120**) abgezogen wird. In einer besonders bevorzugten Ausführungsform des erfindungsgemäßen Verfahrens wird bevorzugt dieser wasserreiche Strom **120** zum Waschen des Stromes **114** eingesetzt. In dieser Ausführungsform wird also letztlich die nach der Vermischung des aus TK stammenden Produktstroms mit wässriger Baselösung und anschließender Phasentrennung erhaltene organische Phase in einem ein- oder mehrstufigen Prozess mit Wasser oder einem mindestens 50 Massen-% Wasser enthaltenden Strom gewaschen. Der am Kopf von **V3** abgezogene Gasstrom (**121**) wird einer Abgasbehandlung, bevorzugt einer Verbrennung, zugeführt.

Der Sumpf der Destillationskolonne **T2** wird bevorzugt teilweise bis vollständig entnommen, teilweise bis vollständig in den Verdampfer **Hv1** geführt (Strom **124**) und dort soweit wie möglich verdampft und wieder in den unteren Teil der Kolonne zurückgeführt. Nicht verdampfbare Anteile werden bevorzugt in Strom **125** abgezogen. Dieser Strom **125** wird entweder einer Entsorgung, bevorzugt Verbrennung, oder Weiterverarbeitung (z. B. zur Gewinnung weiterer Wertprodukte wie des bei der Anilinherstellung als Nebenkomponente anfallenden Diphenylamins), zugeführt.

Eine weitere bevorzugte Ausführungsform des erfindungsgemäßen Verfahrens ist in Figur 2 gezeigt. Es bedeuten:

**Tabelle 2: Bezugszeichen der Figur 2.**

| **Stoffstrom** | **Bedeutung** | **Apparat** | **Bedeutung** |
|---|---|---|---|
| **21** | Gasförmiges rohes Reaktionsprodukt (Roanilin) | **T1** | Wäscher zur Aufteilung des Rohanilinstroms in PK und TK |
| **22** | Aus **T1** ausgetragenes flüssiges Verfahrensprodukt (PK) | **CC1** | Kondensator |
| **23** | Kopfprodukt aus **T1** | **V1** | Abscheider |
| **24** | **23** nach Durchlaufen von **CC1** | **S1** | Splitter |
| **25** | In den Kopf von **T1** zurückgeführter Produktstrom | **C2** | Kühler |
| | | | |
| **251** | In **S1** abgezweigter Produktstrom, der nicht in **T1** zurückgeführt wird | **CC2** | Kondensator |
| **26** | **22** nach Durchlaufen von **C1** | **V2** | Abscheider |
| **261** | **26** nach Vermischung mit **213** (261 = "*Verfahrensprodukt enthaltend PK")* | **C3** | Kühler |
| **27** | Wässrige Baselösung zur Behandlung von PK oder eines Verfahrensproduktes enthaltend PK | **D2** | Dekanter |
| **28** | Kopfprodukt aus V1 | **C1** | Kühler |
| **29** | **28** nach Durchlaufen von **CC2** | **D1** | Dekanter |
| **210** | Kopfprodukt aus **V2** | **H1** | Vorwärmer |
| **211** | Aus **V2** ausgetragenes flüssiges Verfahrensprodukt (TK) | **T2** | Destillationskolonne |
| **2111** | **211** nach Vermischung mit dem Strom aus **C2** | **AT** | Abtriebsteil |
| **212** | Wässrige Baselösung zur Behandlung von TK oder eines Verfahrensproduktes enthaltend TK | **VT1** | Oberes Verstärkungsteil |
| **213** | Wässrige Phase aus **D2** | **VT2** | Mittleres Verstärkungsteil |
| **214** | Organische Phase aus **D2** | **Hv1** | Verdampfer |
| **217** | Organische Phase aus **D1** | **C4** | Kühler |
| **218** | Aus **T2** über Kopf abgezogene Gasphase | **CC3** | Kondensator |
| **219** | **218** nach Durchlaufen von **CC2** | **V3** | Abscheider |
| | | | |
| **220** | Aus **V3** unten abgezogener flüssiger Kondensatstrom | **H2** | Vorwärmer |
| **221** | Kopfprodukt aus **V3** | **T3** | Wasserstripper |
| **222** | Anilinstrom aus Seitenentnahme von **T2** | **Hv2** | Verdampfer |
| **223** | Anilinstrom aus Seitenentnahme von **T2** nach Durchlaufen von **C4** | **C5** | Kühler |
| **224** | Umlauf zum Verdampfer **Hv1** | **CC4** | Kondensator |
| **225** | Ausgeschleuster Bodenablauf aus **T2** | **C5** | Kühler |
| **226** | Wässrige Phase aus **D1** | **CC4** | Kondensator |
| **227** | **226** nach Durchlaufen von **H2** | | |
| **228** | Kopfprodukt aus **T3** (Anilin-Wasser-Azeotrop) | | |
| **229** | Umlaufstrom zum Verdampfer **Hv2** | | |
| **230** | Ausgetragener Bodenablauf aus **T3** | | |
| **231** | **230** nach Durchlaufen von **C5** | | |

Im Unterschied zur in Figur 1 gezeigten Ausführungsform wird hier der aus dem Abscheider **V1** unten abgezogene Strom im Splitter **S1** geteilt und nur teilweise (bevorzugt 5 bis 99 Massen-% des Stromes **9**) in den Kopf von **T1** zurückgeführt (Strom **25**). Der verbleibende Teil (Strom **251**) wird, bevorzugt nach Durchlaufen eines Kühlers **C2**, mit dem als Strom **211** aus **V2** abgezogenen flüssigen Verfahrensprodukt (TK) vermischt, und das so erhaltene Gemisch (Strom **2111**) wird, bevorzugt nach Durchlaufen eines Kühlers **C3**, mit wässriger Baselösung (**212**) behandelt.

Der in **D1** erhaltene wasserreiche Strom **226** wird nicht wie in Figur **1** einer Abwasserbehandlung zugeführt, sondern bevorzugt nach Durchlaufen des Vorwärmers **H2** in den Kopf des Wasserstrippers **T3** geleitet (Strom **227**). Auf diese Weise wird ein Anilin-Wasser-Azeotrop erhalten, welches über Kopf abgezogen (Strom **228**), im Kondensator **CC4** kondensiert und mit Strom **219** vermischt wird. Der Bodenablauf von **T3** wird teilweise über Strom **229** einem Verdampfer **Hv2** zugeführt und dort wieder in den unteren Teil von **T3** zurückgeführt. Verbliebenes Abwasser (Strom **230**) wird nach Durchlaufen des Kühlers **C5** entsorgt, bspw. durch Verbrennung oder in der Kläranlage (Strom **231**). Der Energieeintrag für den Wasserstripper **T3** und die Destillationskolonne **T2** erfolgt bevorzugt mittels Dampf, wobei besonders bevorzugt für **T3** Dampf geringeren Druckes eingesetzt wird als für **T2**.

Der aus TK stammende Produktstrom, der in den Kopf der Destillationskolonne **T2** oberhalb des obersten Verstärkungsteils geleitet wird, ist in dieser Ausführungsform die in **D2** erhaltene organische Phase (Strom **214**). Ein Dekanter **D3** entfällt in dieser Ausführungsform; Strom **220** wird in **D2** geleitet. Strom **214** hat in dieser Ausführungsform bevorzugt eine Temperatur von 30 °C bis 60 °C (analog Strom **216** in Figur 1).

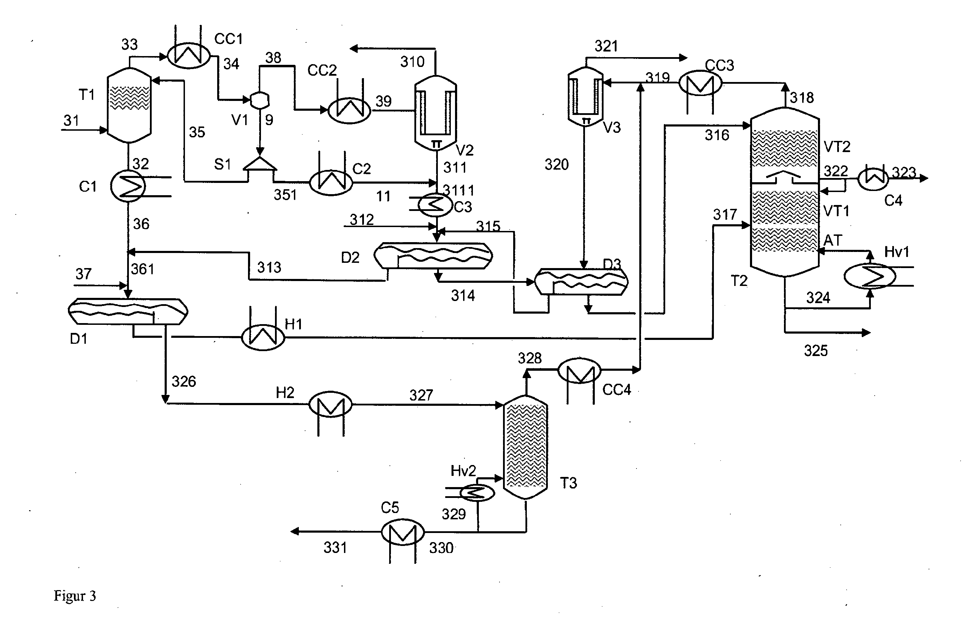

Eine weitere bevorzugte Ausführungsform des erfindungsgemäßen Verfahrens ist in Figur 3 gezeigt. Es bedeuten:

**Tabelle 3: Bezugszeichen der Figur 3.**

| **Stoffstrom** | **Bedeutung** | **Apparat** | **Bedeutung** |
|---|---|---|---|
| **31** | Gasförmiges rohes Reaktionsprodukt (Rohanilin) | **T1** | Wäscher zur Aufteilung des Rohanilinstroms in PK und TK |
| **32** | Aus **T1** ausgetragenes flüssiges Verfahrensprodukt (PK) | **CC1** | Kondensator |
| **33** | Kopfprodukt aus **T1** | **V1** | Abscheider |
| **34** | 33 nach Durchlaufen von CC1 | **S1** | Splitter |
| **35** | In den Kopf von **T1** zurückgeführter Produktstrom | **C2** | Kühler |
| **351** | In **S1** abgezweigter Produktstrom, der nicht in **T1** zurückgeführt wird | **CC2** | Kondensator |
| **36** | **32** nach Durchlaufen von **C1** | **V2** | Abscheider |
| | | | |
| **361** | **36** nach Vermischung mit **313** (361 = *"Verfahrensprodukt enthaltend PK"*) | **C3** | Kühler |
| **37** | Wässrige Baselösung zur Behandlung von PK oder eines Verfahrensproduktes enthaltend PK | **D2** | Dekanter |
| **38** | Kopfprodukt aus **V1** | **C1** | Kühler |
| **39** | **38** nach Durchlaufen von **CC2** | **D1** | Dekanter |
| **310** | Kopfprodukt aus **V2** | **H1** | Vorwärmer |
| **311** | Aus **V2** ausgetragenes flüssiges Verfahrensprodukt (TK) | **D3** | Dekanter |
| **3111** | **311** nach Vermischung mit dem Strom aus **C2** | **T2** | Destillationskolonne |
| **312** | Wässrige Baselösung zur Behandlung von TK oder eines Verfahrensproduktes enthaltend TK | **AT** | Abtriebsteil |
| **313** | Wässrige Phase aus **D2** | **VT1** | Oberes Verstärkungsteil |
| **314** | Organische Phase aus **D2** | **VT2** | Mittleres Verstärkungsteil |
| **315** | Wässrige Phase aus **D3** | **Hv1** | Verdampfer |
| **316** | Organische Phase aus **D3** | **C4** | Kühler |
| **317** | Organische Phase aus **D1** | **CC3** | Kondensator |
| **318** | Aus **T2** über Kopf abgezogene Gasphase | **V3** | Abscheider |
| **319** | **318** nach Durchlaufen von **CC2** | **H2** | Vorwärmer |
| **320** | Aus **V3** unten abgezogener flüssiger Kondensatstrom | **T3** | Wasserstripper |
| **321** | Kopfprodukt aus **V3** | **Hv2** | Verdampfer |
| **322** | Anilinstrom aus Seitenentnahme **von T2** | **C5** | Kühler |
| **323** | Anilinstrom aus Seitenentnahme von **T2** nach Durchlaufen von **C4** | **CC4** | Kondensator |
| **324** | Umlauf zum Verdampfer **Hv1** | | |
| **325** | Ausgeschleuster Bodenablauf aus **T2** | | |
| **326** | Wässrige Phase aus **D1** | | |
| **327** | **326** nach Durchlaufen von **H2** | | |
| **328** | Kopfprodukt aus **T3** (Anilin-Wasser-Azeotrop) | | |
| **329** | Umlaufstrom zum Verdampfer **Hv2** | | |
| **330** | Ausgetragener Bodenablauf aus **T3** | | |
| **331** | **330** nach Durchlaufen von **C5** | | |

Diese Variante ist eine Kombination der in den Figuren 1 und 2 beschriebenen, in welcher der Strom **314** im Dekanter **D3** mit dem Ablauf aus dem Abscheider **V3** (Strom **320**) gewaschen wird. Strom **320** stammt in dieser Verfahrensvariante aus den in **V3** vereinigten Kondensaten aus **CC3** *und* **CC4**. Die Wäsche von Strom **314** kann ohne oder mit Zusatz von Base erfolgen, bevorzugt ohne. Im Fall einer Wäsche unter Zusatz von Base (in Figur 3 nicht gezeigt) wird bevorzugt die gleiche Base wie bei der Basebehandlung von Strom **32** bzw. (in der bevorzugten Ausführungsform) Strom **36** eingesetzt, jedoch bevorzugt in erheblich geringerer Menge und Konzentration.

Figur 4 zeigt eine Ausführungsform des erfindungsgemäßen Verfahrens, in der die Basebehandlung *nach* der Destillation erfolgt. Es bedeuten:

**Tabelle 4: Bezugszeichen der Figur 4.**

| **Stoffstrom** | **Bedeutung** | **Apparat** | **Bedeutung** |
|---|---|---|---|
| **41** | Gasförmiges rohes Reaktionsprodukt (Rohanilin) | **T1** | Wäscher zur Aufteilung des Rohanilinstroms in PK und TK |
| **42** | Aus **T1** ausgetragenes flüssiges Verfahrensprodukt (PK) | **CC1** | Kondensator |
| **43** | Kopfprodukt aus **T1** | **V1** | Abscheider |
| **44** | **43** nach Durchlaufen von **CC1** | **S1** | Splitter |
| **45** | In den Kopf von **T1** zurückgeführter Produktstrom | **C2** | Kühler |
| **451** | In **S1** abgezweigter Produktstrom, der nicht in **T1** zurückgeführt wird | **CC2** | Kondensator |
| **48** | Kopfprodukt aus V1 | **V2** | Abscheider |
| **49** | **48** nach Durchlaufen von **CC2** | **C3** | Kühler |
| **410** | Kopfprodukt aus **V2** | **D2** | Dekanter |
| **411** | Aus **V2** ausgetragenes flüssiges Verfahrensprodukt (TK) | **T2** | Destillationskolonne |
| **4111** | **411** nach Vermischung mit dem Strom aus **C2** | **AT** | Abtriebsteil |
| **41111** | **4111** nach Vermischung mit **420** | **VT1** | Oberes Verstärkungsteil |
| **413** | Wässrige Phase aus **D2** | **VT2** | Mittleres Verstärkungsteil |
| **414** | Organische Phase aus **D2** | **Hv1** | Verdampfer |
| **418** | Aus **T2** über Kopf abgezogene Gasphase | **V3** | Abscheider |
| | | | |
| **420** | Aus **V3** unten abgezogener flüssiger Kondensatstrom | **C5** | Kühler |
| **421** | Kopfprodukt aus **V3** | **D4** | Dekanter |
| **422** | Anilinstrom aus Seitenentnahme von **T2** | **D5** | Dekanter |
| **424** | Umlaufstrom zum Verdampfer **Hv1** | **CC4** | Kondensator |
| **425** | Ausgeschleuster Bodenablauf aus **T2** | **T3** | Wasserstripper |
| **428** | Kopfprodukt aus **T3** (Anilin-Wasser-Azeotrop) | **Hv2** | Verdampfer |
| **429** | Umlaufstrom zum Verdampfer **Hv2** | **C7** | Kühler |
| **430** | Ausgetragener Bodenablauf aus **T3** | | |
| **432** | Wässrige Baselösung | | |
| **433** | Organische Phase aus **D4** | | |
| **434** | Organische Phase aus **D5** (= wasserhaltiges gereinigtes Anilin) | | |
| **435** | Wässrige Phase aus **D4** | | |
| **436** | Wässrige Phase aus **D5** | | |

Der aus PK stammende Produktstrom, der in den unteren Teil der Destillationskolonne **T2** zwischen dem untersten Abtriebsteil und der darauf folgenden Sektion eingeleitet wird, ist in dieser Ausführungsform identisch mit PK selbst, d. h. ist das als Strom **42** aus **T1** ausgetragene flüssige Verfahrensprodukt. Der aus TK stammende Produktstrom, der in den Kopf der Destillationskolonne **T2** oberhalb des obersten Verstärkungsteils geleitet wird, ist in dieser Ausführungsform wie in Figur 2 die in **D2** erhaltene organische Phase (Strom **414**). In dieser Variante der Erfindung wird die wässrige Phase aus **D2** (Strom **413**) teilweise bis vollständig in einen Wasserstripper (**T3**) zur Gewinnung eines Anilin-Wasser-Azeotrops (hier **428**) geleitet. Das Anilin-Wasser-Azeotrop **428** wird nach Durchlaufen des Kondensators **CC4** zum Waschen des Totalkondensats im Dekanter **D2** genutzt.

Der Bodenablauf von **V3** (Strom **420**) wird in dieser Ausführungsform bevorzugt mit Strom **4111** vermischt. Das so erhaltene Gemisch (Strom **41111**) wird wie zuvor beschrieben in **D2** geleitet.

Das destillierte Anilin **422** wird mit wässriger Baselösung **432** versetzt. Das so erhaltene Verfahrensprodukt wird optional mit wässriger Phase aus **D2** (Strom **413**) vermischt und dann nach Durchlaufen eines Kühler **C5** in den Dekanter **D4** geleitet, wo die aciden Verunreinigungen bei Temperaturen von 80 °C - 160 °C neutralisiert werden. Die wässrige Phase aus **D4** (Strom **437**) wird in den Kopf von **T3** zurückgeführt Die organische, noch stark wasserhaltige Phase aus **D4** (Strom **433**) wird über einen Kühler **C7** in einen weiteren Dekanter **D5** geleitet und bei 20 °C bis 40 °C einer Phasentrennung unterworfen, um den Wassergehalt des gereinigten Anilins (Strom **434**) soweit wie möglich zu reduzieren. Das gereinigte Anilin **434** enthält im Wesentlichen nur noch Wasser, entsprechend der Löslichkeit von Wasser in Anilin bei der Temperatur im Dekanter **D5**. Verbindungen mit phenolischen Hydroxygruppen und andere Nebenkomponenten sind nur in unwesentlichen Mengen vorhanden (in Summe < 100 ppm, bevorzugt < 50 ppm, besonders bevorzugt < 25 ppm, bezogen auf die Masse des organischen Anteils des Stromes **434**) Abhängig von der gewünschten Verwendung des Anilin kann dieses entweder so verbleiben oder getrocknet werden, bevorzugt durch Strippung (nicht gezeigt in Figur 4).

Die wässrige Phase aus **D5** (Strom **436**) wird ebenfalls in den Kopf von **T3** zurückgeführt.

Das erfindungsgemäße Verfahren zeichnet sich gegenüber dem Stand der Technik dadurch aus, dass nur *ein Teil* des gereinigten Anilins die Schritte (i) Kondensation - (ii) Verdampfung - (iii) erneute Kondensation durchlaufen hat und nicht wie im Stand der Technik üblich das *gesamte* gereinigte Anilin. Im erfindungsgemäßen Verfahren hat nämlich nur derjenige Anteil des gereinigten Anilins, der aus PK stammt, die Schritte (i) bis (iii) komplett durchlaufen. Derjenige Anteil des gereinigten Anilins jedoch, der aus TK stammt, hat die Schritte (i) bis (iii) *nur zum Teil* durchlaufen. Der Anteil des im in den Kopf der Kolonne oberhalb des obersten Verstärkungsteils eingeleiteten, aus TK stammenden Produktstrom enthaltenen Anilins, welcher direkt, also nicht über die Ströme **118/218/318 -119/219/319 - 120/220/320** bzw. **418/420/4111**, in den Reinanilinstrom gelangt, wird nämlich nach der Kondensation (i) *nicht wieder verdampft.* Dieser Anteil an Anilin, der nicht wieder verdampft wird, beträgt bevorzugt, abhängig von den genauen Randbedingungen, zwischen 65 Massen-% und 99,9 Massen-% der Gesamtmasse des Reinanilinstromes (**123/223/323** bzw. **434** nach Trocknung). Hierdurch werden der energetische und apparative Aufwand sowie die thermische Belastung des wertvollen Anilins signifikant verringert.

Ferner gelingt, falls es die jeweilige Trennaufgabe erfordert, durch die geschilderte Basenbehandlung in Kombination mit der geschilderten Destillation auf einfache Weise eine effektive Reduzierung des Gehalts an Verbindungen mit phenolischen Hydroxygruppen im gereinigten Anilin.

### Beispiele

In den folgenden Beispielen wird die Reinigung eines Rohanilins beschrieben, welches mit einem Massenstrom von 35 000 kg / h und einer Temperatur von 147 °C einer Produktionsanlage entströmt und wie folgt zusammengesetzt ist:

**Tabelle 5: Zusammensetzung des zu reinigenden Rohanilinstromes.**

| **Komponente** | **Massenanteil in %** |
|---|---|
| Nicht kondensierbare Gase | 21,46 |
| Leichtsieder | 1,140 |
| Wasser | 25,75 |
| Anilin | 51,51 |
| Phenol | 0,04 |
| Hochsieder | 0,1 |

In allen Fällen wurde als wässrige Baselösung 32%ige Natronlauge eingesetzt, und zwar in stöchiometrischer Menge in Bezug auf Phenol. Die Destillation erfolgt jeweils mit einer aus drei Sektionen (von unten nach oben: unteres Abtriebsteil (**AT**), mittleres Verstärkungsteil (**VT1**), oberes Verstärkungsteil (**VT2**)) bestehenden Kolonne, die über einen Verdampfer **Hv2** verfügt.

### Beispiel 1 (Vergleichsbeispiel: ein Kondensationsschritt, Base ist während der Destillation vorhanden. ASPEN-Simulation)

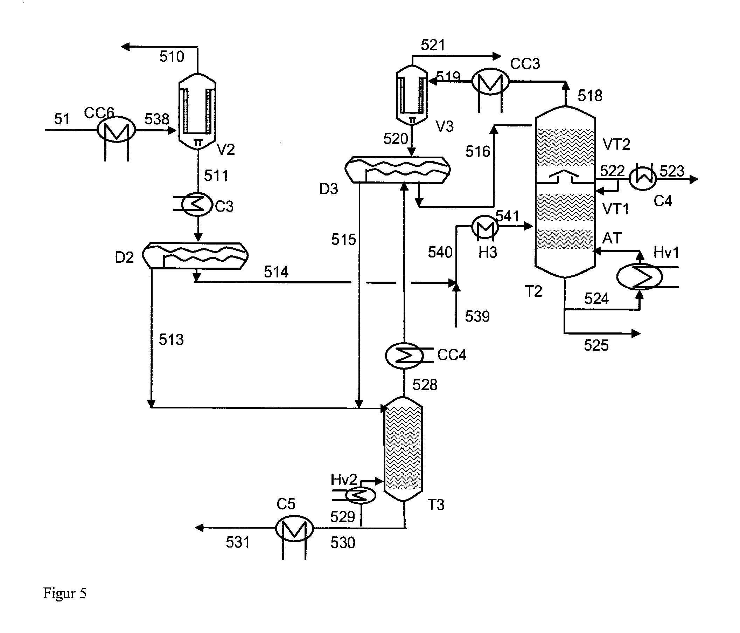

Figur 5 zeigt den in diesem Beispiel angewandten Prozess. Es bedeuten:

**Tabelle 6: Bezugszeichen der Figur 5.**

| **Stoffstrom** | **Bedeutung** | **Apparat** | **Bedeutung** |
|---|---|---|---|
| **51** | Gasförmiges rohes Reaktionsprodukt (Rohanilin) | **CC6** | Kondensator |
| **510** | Kopfprodukt aus **V2** | **V2** | Abscheider |
| **511** | Bodenablauf aus **V2** | **C3** | Kühler |
| **513** | Wässrige Phase aus **D2** | **D2** | Dekanter |
| **514** | Organische Phase aus **D2** | **H3** | Vorwärmer |
| **515** | Wässrige Phase aus **D3** | **T2** | Destillationskolonne |
| **516** | Organische Phase aus **D3** | **AT** | Abtriebsteil |
| **518** | Aus **T2** über Kopf abgezogene Gasphase | **VT1** | Oberes Verstärkungsteil |
| **519** | Kopfprodukt aus **T2** nach Durchlaufen von **CC2** | **VT2** | Mittleres Verstärkungsteil |
| **520** | Bodenablauf aus **V3** | **Hv1** | Verdampfer |
| **521** | Kopfprodukt aus **V3** | **C4** | Kühler |
| **522** | Anilinstrom aus Seitenentnahme von **T2** | **CC3** | Kondensator |
| **523** | Anilinstrom aus Seitenentnahme von **T2** nach Durchlaufen von **C4** | **V3** | Abscheider |
| **524** | Umlauf zum Verdampfer **Hv1** | **D3** | Dekanter |
| **525** | Ausgeschleuster Bodenablauf aus **T2** | **T3** | Wasserstripper |
| **528** | Kopfprodukt aus **T3** (Anilin-Wasser-Azeotrop) | **Hv2** | Verdampfer |
| **529** | Umlauf zum Verdampfer **Hv2** | **C5** | Kühler |
| **530** | Ausgetragener Bodenablauf aus **T3** | **CC4** | Kondensator |
| **531** | **330** nach Durchlaufen von **C5** | | |
| **538** | **51** nach Durchlaufen von **CC6** | | |
| **539** | Wässrige Baselösung | | |
| **540** | Vereinigte Ströme **514** und **539** | | |
| **541** | **540** nach Durchlaufen von **H3** | | |

Folgende Bedingungen wurden zugrundegelegt:

| | |
|---|---|
| Temperatur des Gases nach **CC6** (Strom **538**): | 60 °C |
| Zahl der theoretischen Stufen des oberen Verstärkungsteils von **T2:** | 10 |
| Zahl der theoretischen Stufen des mittleren Verstärkungsteils von **T2:** | 9 |
| Zahl der theoretischen Stufen des unteren Abtriebsteils von **T2:** | 12 |

Bei dieser Verfahrensführung wird der Rohanilinstrom **51** nicht fraktionierend kondensiert, sondern im Kondensator **CC2** in einem Schritt möglichst vollständig kondensiert (mehr als 95 % des Anilins). Das so erhaltene Verfahrensprodukt (**511** - das "Totalkondensat" dieses Verfahrens) wird zur Rückführung der nicht kondensierten Anteile in den Hydrierprozess (via Strom **510**) in einen Abscheider **V2** geleitet. Das gesamte kondensierte Rohprodukt wird in **C3** weiter gekühlt und dann einer Phasentrennung im Dekanter **D2** unterworfen. Die Zuführung von Natronlauge (**539**) erfolgt zu der so erhaltenen organischen Phase **514**. Das so erhaltene, NaOH-haltige Verfahrensprodukt **540** wird nach Durchlaufen eines Vorwärmers **H3** zwischen **AT** und **VT1** in die Destillationskolonne **T2** geleitet (Strom **541**). Anilin (Strom **522**) wird als Seitenstrom zwischen **VT2** und **VT2** entnommen und teilweise wieder in die Kolonne zurückgeführt. Der nicht zurückgeführte Anteil wird unter Dampfgewinnung in **C4** gekühlt und als Reinanilinstrom **523** entnommen. Der Massenfluss des Sumpfproduktes, Strom **524**, wird so eingestellt, dass im Sumpf von **T2** keine Phenolatsalze ausfallen. Die in **T2** abgeführte Gasphase, Strom **518**, wird in **CC3** auf 55 °C gekühlt und in eine Vorrichtung zur Trennung von Gasen und Flüssigkeiten (**V3**) geleitet. Das Abgas (Strom **521**) wird der Verbrennung zugeführt. Die Flüssigphase von **V3** wird im Dekanter **D3** in eine anilinreiche und eine wasserreiche Phase getrennt. Die anilinreiche Phase wird in den Kopf der Kolonne **T2** geführt (**516**). Die wasserreichen Phasen aus **D2** (Strom **13**) und **D3** (Strom **15**) werden in einen Wasserstripper (**T3**) geleitet, der sie in einen Wasserstrom mit weniger als 100 ppm Anilin (**529**)) und ein Wasser-Anilin-Azeotrop (**528**) trennt. Letzteres wird in **CC4**'(**V**) kondensiert und nach **D3**(**V**) geführt. Die Ergebnisse sind in Tabelle 2 zusammengefasst:

**Tabelle 7: Zusammensetzungen der Ströme und nötiger Energieeintrag in Beispiel 1.**

| | **Strom^{[a]}** | | |
|---|---|---|---|
| | **510** | **523** | **525** |
| Gesamtmassenstrom | 10506 kg/h | 16935 kg/h | 736 kg/h |
| Nichtkondensierbare Gase | 71,42 % | 0,00 % | 0,00 % |
| Leichtsieder | 3,29 % | 75,4 ppm | 0,0 ppm |
| Wasser | 21,29 % | 0,09 % | 0,00 % |
| Anilin | 4,00 % | 99,90 % | 93,42 % |
| Summe aus Phenol und Natriumphenolat | 9,1 ppm | 0,0 ppm | 2,50 % |
| Hochsieder | 0,00 % | 0,0 ppm | 4,08 % |
| Temperatur | 60 °C | 40 °C | 144 °C |
| | | | |
| Für **T2** nötiger Energieeintrag | 5125 kW | | |
| Für **T3** nötiger Energieeintrag | 3103 kW | | |

| | | | |
|---|---|---|---|
| [a] Gehaltsangaben in % und ppm sind stets Massenanteile. | | | |

Wie man sieht, involviert diese Vorgehensweise die erneute Verdampfung *des gesamten Anilins,* um es von den Hochsiedern abzutrennen. Auch müssen signifikante Anilinverluste über Strom **525** in Kauf genommen werden, um das Ausfallen von Phenolatsalzen in **Hv1** zu vermeiden. Der Energieeintrag in **T2** ist höher als der in **T3**. Dies ist besonders nachteilig, weil für das Betreiben von **T2** Dampf höheren Drucks erforderlich ist als für das Betreiben von **T3**. Der gesamte für **T2** und **T3** erforderliche Energieeintrag ist mit 8228 kW sehr hoch.

### Beispiel 2 (erfindungsgemäß: zwei Kondensationsschritte, jeweils eine basische Extraktion vor der Destillation. ASPEN-Simulation

Diese Simulation wurde anhand der in Figur 2 und weiter oben bereits näher erläuterten Verfahrensvariante durchgeführt. 45 % der in **CC1** kondensierten Flüssigkeit werden als Strom **5** in den Wäscher **T1** geleitet. Folgende Bedingungen wurden zugrunde gelegt:

| | |
|---|---|
| Temperatur des Gases nach **CC1** (Strom **24**): | 118 °C |
| Zahl der theoretischen Stufen in **T1:** | 6 |
| Zahl der theoretischen Stufen des oberen Verstärkungsteils von **T2:** | 10 |
| Zahl der theoretischen Stufen des mittleren Verstärkungsteils von **T2:** | 10 |
| Zahl der theoretischen Stufen des unteren Abtriebsteils von **T2:** | 11 |
| Temperatur in **CC2:** | 60 °C |

Die Ergebnisse sind in Tabelle 3 zusammengefasst.

**Tabelle 8: Zusammensetzungen der Ströme und nötiger Energieeintrag in Beispiel 2.**

| | **Strom^{[a]}** | | |
|---|---|---|---|
| | **210** | **223** | **225** |
| Gesamtmassenstrom | 9980 kg/h | 17679 kg/h | 62 kg/h |
| Nichtkondensierbare Gase | 75,17 % | 0,00 % | 0,00 % |
| Leichtsieder | 3,80 % | 20,2 ppm | 0,0 ppm |
| Wasser | 17,71 % | 0,09 % | 0,00 % |
| Anilin | 3,32 % | 99,90 % | 51,42 % |
| Summe aus Phenol und Natriumphenolat | 9,1 ppm | 4,2 ppm | 0,11 % |
| Hochsieder | 0,00 % | 1,7 ppm | 48,47 % |
| Temperatur | 60 °C | 40 °C | 156 °C |
| | | | |
| Für **T2** nötiger Energieeintrag | 1673 kW | | |
| Für **T3** nötiger Energieeintrag | 3251 kW | | |

| | | | |
|---|---|---|---|
| [a] Gehaltsangaben in % und ppm sind stets Massenanteile. | | | |

Wie man sieht, führt die Anwendung des erfindungsgemäßen Verfahrens zu einer deutlichen Reduktion sowohl des Energieverbrauchs in **T2** als auch der Anilinverluste über Strom **225**. Der Gesamtenergieverbrauch in **T2** und **T3** beträgt nur noch 4924 kW gegenüber 8228 kW im Vergleichsbeispiel. Die Produktqualität unterscheidet sich nur dadurch, dass Spuren an Phenolatsalzen im Produkt auftreten können, jedoch noch in akzeptabler Menge. Das folgende Beispiel zeigt, wie diese Spuren auf einen insignifikanten Wert reduziert werden können.

### Beispiel 3 (erfindungsgemäß: zwei Kondensationsschritte, jeweils eine basische Extraktion und eine neutrale Wäsche vor der Destillation. ASPEN-Simulation

Diese Simulation wurde anhand der in Figur 3 beschriebenen und weiter oben bereits erläuterten Verfahrensvariante durchgeführt. Die übrigen Bedingungen sind die gleichen wie in Beispiel 2. Die Ergebnisse sind in Tabelle 4 zusammengefasst.

**Tabelle 9: Zusammensetzungen der Ströme und nötiger Energieeintrag in Beispiel 3.**

| | **Strom^{[a]}** | | |
|---|---|---|---|
| | **310** | **323** | **325** |
| Gesamtmassenstrom | 9980 kg/h | 17679 kg/h | 62 kg/h |
| Nichtkondensierbare Gase | 75,17 % | 0,00 % | 0,00 % |
| Leichtsieder | 3,80 % | 19,0 ppm | 0,0 ppm |
| Wasser | 17,71 % | 0,09 % | 0,00 % |
| Anilin | 3,32 % | 99,90 % | 51,44 % |
| Summe aus Phenol und Natriumphenolat | 9,1 ppm | 0,1 ppm | 0,10 % |
| Hochsieder | 0,00 % | 1,7 ppm | 48,47 % |
| Temperatur | 60 °C | 40 °C | 156 °C |
| | | | |
| Für **T2** nötiger Energieeintrag | 1659 kW | | |
| Für **T3** nötiger Energieeintrag | 3332 kW | | |

| | | | |
|---|---|---|---|
| [a] Gehaltsangaben in % und ppm sind stets Massenanteile. | | | |

Wie man sieht, ist der Reinanilinstrom **323** nun praktisch frei von Phenol- bzw.- Phenolatverunreinigungen. Der Gesamtenergieverbrauch in **T2** und **T3** beträgt nur noch 4991 kW gegenüber 8228 kW im Vergleichsbeispiel.

### Beispiel 4 (erfindungsgemäß: zwei Kondensationsschritte, jeweils eine basische Extraktion und eine neutrale Wäsche vor der Destillation, kleinere Destillationskolonne. ASPEN-Simulation

Diese Simulation wurde ebenfalls anhand des in Figur 3 beschriebenen Verfahrens durchgeführt. In diesem Beispiel hat die Kolonne **T2** weniger Stufen.

| | |
|---|---|
| Zahl der theoretischen Stufen des oberen Verstärkungsteils von **T2:** | 9 |
| Zahl der theoretischen Stufen des mittleren Verstärkungsteils von **T2:** | 6 |
| Zahl der theoretischen Stufen des unteren Abtriebsteils von **T2:** | 2 |

Die übrigen Parameter sind die gleichen wie in Beispiel 3.

Der Hintergrund dieses Beispiels ist es aufzuzeigen, dass die Anzahl der Stufen in **T2** eine optimierbare Variable ist. Das erfindungsgemäße Verfahren erlaubt in besonderer Weise eine Reduktion der Stufen und damit auch eine Senkung von Produktions- und Fixkosten.

Die Ergebnisse sind in Tabelle 5 zusammengefasst.

**Tabelle 10: Zusammensetzungen der Ströme und nötiger Energieeintrag in Beispiel 4.**

| | **Strom^{[a]}** | | |
|---|---|---|---|
| | **310** | **323** | **325** |
| Gesamtmassenstrom | 9980 kg/h | 17679 kg/h | 62 kgh |
| Nichtkondensierbare Gase | 75,17 % | 0,00 % | 0,00 % |
| Leichtsieder | 3,80 % | 19 ppm | 0,5 ppm |
| Wasser | 17,71 % | 0,09 % | 0,00 % |
| Anilin | 3,32 % | 99,90 % | 51,46 % |
| Summe aus Phenol und Natriumphenolat | 9,1 ppm | 0,1 ppm | 0,09 % |
| Hochsieder | 0,00 % | 0,8 ppm | 48,44 % |
| Temperatur | 60 °C | 40 °C | 156 °C |
| | | | |
| Für **T2** nötiger Energieeintrag | 1659 kW | | |
| Für **T3** nötiger Energieeintrag | 3333 kW | | |

| | | | |
|---|---|---|---|
| [a] Gehaltsangaben in % und ppm sind stets Massenanteile. | | | |

Wie man sieht, ist die geringere Stufenzahl völlig ausreichend. Der Hochsiedergehalt im Reinanilinstrom **323** ist gegenüber Beispiel 3 sogar noch verringert. Der Gesamtenergieverbrauch in **T2** und **T3** beträgt nur noch 4992 kW gegenüber 8228 kW im Vergleichsbeispiel.

## Patentansprüche

1. Verfahren zur Herstellung von Anilin durch Gasphasenhydrierung von Nitrobenzol und anschließende Reinigung des Anilins, worin
ein gasförmiges rohes Reaktionsprodukt (**11/21/31/41**) anfällt, welches fraktionierend kondensiert wird, sodass mindestens zwei flüssige Verfahrensprodukte, wenigstens ein Partialkondensat (PK) (**12/22/32/42**) und ein Totalkondensat (**TK**) (**111/211/311/411**) erhalten werden, wobei
die zur Bildung von PK (**12/22/32/42**) führende Kondensation bei höherer Temperatur durchgeführt wird als die zur Bildung von TK (**111/211/311/411**) führende Kondensation, **dadurch gekennzeichnet, dass**
ein aus PK stammender Produktstrom und ein aus TK stammender Produktstrom in eine Destillationskolonne (**T2**) umfassend mindestens zwei Sektionen, nämlich mindestens ein Abtriebsteil und mindestens ein Verstärkungsteil, geleitet werden,
wobei der aus PK stammende Produktstrom in den unteren Teil der Destillationskolonne zwischen dem untersten Abtriebsteil und der darauf folgenden Sektion und der aus TK stammende Produktstrom in den Kopf der Destillationskolonne oberhalb des obersten Verstärkungsteils eingeleitet werden, und wobei
destilliertes Anilin (**122/222/322/422**) in einem Seitenstrom zwischen unterstem Abtriebsteil und oberstem Verstärkungsteil der Destillationskolonne entnommen wird.

2. Verfahren nach Anspruch 1, wobei
die zur Bildung von PK (**12/22/32/42**) führende Kondensation bei Temperaturen von 60 °C bis 160 °C und
die zur Bildung von TK (**111/211/311/411**) führende Kondensation bei Temperaturen von 25 °C bis 90 °C
durchgeführt wird.

3. Verfahren nach Anspruch 1 oder 2, wobei
der aus PK stammende Produktstrom ein in der fraktionierenden Kondensation erhaltenes Partialkondensat (**42**) ist und
der aus TK stammende Produktstrom eine organische Phase (**414**) ist, die durch Phasentrennung von TK (**411**) oder eines Verfahrensproduktes enthaltend TK (**4111/41111**) in eine wässrige und eine organische Phase erhalten wird.

4. Verfahren nach Anspruch 1 oder 2, wobei
der aus PK stammende Produktstrom eine organische Phase (**117/217/317**) ist, die nach Vermischung von PK (**12/22/32**) oder eines Verfahrensproduktes enthaltend PK (**161/261/361**) mit wässriger Baselösung (**17/27/37**) und anschließender Phasentrennung in eine wässrige und eine organische Phase erhalten wird und
der aus TK stammende Produktstrom eine organische Phase (**116/214/316**) ist, die nach Vermischung von TK (**111/211/311**) oder eines Verfahrensproduktes enthaltend TK (**2111/3111**) mit wässriger Baselösung (**112/212/312**) und anschließender Phasentrennung in eine wässrige und eine organische Phase erhalten wird.

5. Verfahren nach Anspruch 4, wobei
PK (**12/22/32**) oder das Verfahrensprodukt enthaltend PK (**161/261/361**) vor, während oder nach der Vermischung mit wässriger Baselösung (**17/27/37**) in einem ein- oder mehrstufigen Prozess mit Wasser oder einem mindestens 50 Massen-% Wasser enthaltenden Strom A, bezogen auf die Gesamtmasse dieses Stromes A, vermischt wird.

6. Verfahren nach Anspruch 5, wobei
der mindestens 50 Massen-% Wasser enthaltende Strom A eine wässrige Phase (**113/213/313**) ist, die nach Vermischung von TK (**111/211/311**) oder eines Verfahrensproduktes enthaltend TK (**2111/3111**) mit wässriger Baselösung (**112/212/312**) und anschließender Phasentrennung in eine wässrige und eine organische Phase erhalten wird.

7. Verfahren nach einem der Ansprüche 4 bis 6, wobei
die nach der Vermischung von TK (**111/311**) oder eines Verfahrensproduktes enthaltend TK (**3111**) mit wässriger Baselösung (**112/312**) und anschließender Phasentrennung erhaltene organische Phase (**114/314**) in einem ein- oder mehrstufigen Prozess mit Wasser oder einem mindestens 50 Massen-% Wasser enthaltenden Strom B, bezogen auf die Gesamtmasse dieses Stromes B, gewaschen wird.

8. Verfahren nach Anspruch 7, wobei
der mindestens 50 Massen-% Wasser enthaltende Strom B ein flüssiger Kondensatstrom (**120**) der über Kopf der Destillationskolonne (**T2**) abgezogenen Gasphase (**118**) oder ein einen flüssigen Kondensatstrom der über Kopf der Destillationskolonne (**T2**) abgezogenen Gasphase (**318**) enthaltendes Verfahrensprodukt (**320**) ist.

9. Verfahren nach einem der Ansprüche 4 bis 6, wobei
TK (**211**) oder das Verfahrensprodukt enthaltend TK (**2111**) vor, während oder nach der Vermischung mit wässriger Baselösung (**212**) in einem ein- oder mehrstufigen Prozess mit Wasser oder einem mindestens 50 Massen-% Wasser enthaltenden Strom B, bezogen auf die Gesamtmasse dieses Stromes B, vermischt wird.

10. Verfahren nach Anspruch 9, wobei
der mindestens 50 Massen-% Wasser enthaltende Strom B ein den flüssigen Kondensatstrom der über Kopf der Destillationskolonne (**T2**) abgezogenen Gasphase (**218**) enthaltendes Verfahrensprodukt (**220**) ist.

11. Verfahren nach Anspruch 8, wobei
das den flüssigen Kondensatstrom der über Kopf der Destillationskolonne (**T2**) abgezogenen Gasphase enthaltende Verfahrensprodukt (**320**)
durch Zugabe eines Anilin-Wasser-Azeotrops (**328**),
erhalten durch Einleitung einer durch Phasentrennung nach der Vermischung von PK (**32**) oder eines Verfahrensproduktes enthaltend PK (**313**) mit wässriger Baselösung (**37**) erhaltenen wässrigen Phase (**326**) in einen Wasserstripper (**T3**),
zum Gesamtkondensat (**319**) der über Kopf der Destillationskolonne (**T2**) abgezogenen Gasphase (**318**)
erhalten wird.

12. Verfahren nach Anspruch 10, wobei
das den flüssigen Kondensatstrom der über Kopf der Destillationskolonne (**T2**) abgezogenen Gasphase enthaltende Verfahrensprodukt (**220**)
durch Zugabe eines Anilin-Wasser-Azeotrops (**228**),
erhalten durch Einleitung einer durch Phasentrennung nach der Verwischung von PK (**22**) oder eines Verfahrensproduktes enthaltend PK (**213**) mit wässriger Baselösung (**27**) erhaltenen wässrigen Phase (**226**) in einen Wasserstripper (**T3**),
zum Gesamtkondensat (**219**) der über Kopf der Destillationskolonne (**T2**) abgezogenen Gasphase (**218**)
erhalten wird

13. Verfahren nach Anspruch 3, wobei das der Destillationskolonne (**T2**) in einem Seitenstrom entnommene Anilin (**422**) mit wässriger Baselösung (**432**) vermischt und ein an sauren Verunreinigungen abgereichertes Anilin (**434**) durch eine Phasentrennung gewonnen wird.

14. Verfahren nach einem der Ansprüche 4 bis 11, wobei als wässrige Baselösung (**17/27/37-112/212/312 - 432**) eine Lösung eines Alkalimetallhydroxids oder Erdalkalimetallhydroxids eingesetzt wird.

## Claims

1. Process for the production of aniline by gas-phase hydrogenation of nitrobenzene and subsequent purification of the aniline, wherein
there is obtained a gaseous crude reaction product (**11/21/31/41**), which is subjected to fractional condensation so that at least two liquid process products, at least one partial condensate (PK) (**12/22/32/42**) and a total condensate (TK) (**111/211/311/411**), are obtained, wherein
the condensation leading to the formation of PK (**12/22/32/42**) is carried out at a higher temperature than the condensation leading to the formation of TK (**111/211/311/411**), **characterized in that**
a product stream originating from PK and a product stream originating from TK are passed into a distillation column (**T2**) comprising at least two sections, namely at least one stripping section and at least one rectifying section,
wherein the product stream originating from PK is introduced into the lower section of the distillation column between the lowermost stripping section and the section which follows, and the product stream originating from TK is introduced into the top of the distillation column above the uppermost rectifying section, and wherein
distilled aniline (**122/222/322/422**) is withdrawn in a side stream between the lowermost stripping section and the uppermost rectifying section of the distillation column.

2. Process according to Claim 1, wherein
the condensation leading to the formation of PK (**12/22/32/42**) is carried out at temperatures of from 60°C to 160°C, and
the condensation leading to the formation of TK (**111/211/311/411**) is carried out at temperatures of from 25°C to 90°C.

3. Process according to Claim 1 or 2, wherein
the product stream originating from PK is a partial condensate (**42**) obtained in the fractional condensation, and
the product stream originating from TK is an organic phase (**414**) which is obtained by phase separation of TK (**411**) or a process product comprising TK (**4111/41111**) into an aqueous phase and an organic phase.

4. Process according to Claim 1 or 2, wherein
the product stream originating from PK is an organic phase (**117/217/317**) which is obtained after mixing PK (**12/22/32**) or a process product comprising PK (**161/261/361**) with aqueous base solution (**17/27/37**) and subsequent phase separation into an aqueous phase and an organic phase, and
the product stream originating from TK is an organic phase (**116/214/316**) which is obtained after mixing TK (**111/211/311**) or a process product comprising TK (**2111/3111**) with aqueous base solution (**112/212/312**) and subsequent phase separation into an aqueous phase and an organic phase.

5. Process according to Claim 4, wherein
PK (**12/22/32**) or the process product comprising PK (**161/261/361**) is mixed, before, during or after mixing with aqueous base solution (**17/27/37**), in a single- or multi-stage process with water or with a stream A comprising at least 50% by mass water, based on the total mass of said stream A.

6. Process according to Claim 5, wherein
stream A comprising at least 50% by mass water is an aqueous phase (**113/213/313**) which is obtained after mixing TK (**111/211/311**) or a process product comprising TK (**2111/3111**) with aqueous base solution (**112/212/312**) and subsequent phase separation into an aqueous phase and an organic phase.

7. Process according to any one of Claims 4 to 6, wherein
the organic phase (**114/314**) obtained after mixing TK (**111/311**) or a process product comprising TK (**3111**) with aqueous base solution (**112/312**) and subsequent phase separation is washed in a single- or multi-stage process with water or with a stream B comprising at least 50% by mass water, based on the total mass of said stream B.

8. Process according to Claim 7, wherein
stream B comprising at least 50% by mass water is a liquid condensate stream (**120**) of the gas phase (**118**) removed at the top of the distillation column (**T2**) or a process product (**320**) comprising a liquid condensate stream of the gas phase (**318**) removed at the top of the distillation column (**T2**).

9. Process according to any one of Claims 4 to 6, wherein
TK (**211**) or the process product comprising TK (**2111**) is mixed, before, during or after mixing with aqueous base solution (**212**), in a single- or multi-stage process with water or with a stream B comprising at least 50% by mass water, based on the total mass of said stream B.

10. Process according to Claim 9, wherein
stream B comprising at least 50% by mass water is a process product (**220**) comprising the liquid condensate stream of the gas phase (**218**) removed at the top of the distillation column (**T2**).

11. Process according to Claim 8, wherein
the process product (**320**) comprising the liquid condensate stream of the gas phase removed at the top of the distillation column (**T2**) is obtained
by adding an aniline-water azeotrope (**328**),
obtained by introducing into a water stripper (**T3**) an aqueous phase (**326**) obtained by phase separation after mixing PK (**32**) or a process product comprising PK (**313**) with aqueous base solution (**37**),
to the total condensate (**319**) of the gas phase (**318**) removed at the top of the distillation column (**T2**).

12. Process according to Claim 10, wherein
the process product (**220**) comprising the liquid condensate stream of the gas phase removed at the top of the distillation column (**T2**) is obtained
by adding an aniline-water azeotrope (**228**),
obtained by introducing into a water stripper (**T3**) an aqueous phase (**226**) obtained by phase separation after mixing PK (**22**) or a process product comprising PK (**213**) with aqueous base solution (**27**),
to the total condensate (**219**) of the gas phase (**218**) removed at the top of the distillation column (**T2**).

13. Process according to Claim 3, wherein the aniline (**422**) withdrawn from the distillation column (**T2**) in a side stream is mixed with aqueous base solution (**432**), and an aniline (**434**) depleted of acidic impurities is obtained by a phase separation.

14. Process according to any one of Claims 4 to 11, wherein there is used as the aqueous base solution (**17/27/37 - 112/212/312 - 432**) a solution of an alkali metal hydroxide or alkaline earth metal hydroxide.

## Revendications

1. Procédé pour la préparation d'aniline par hydrogénation en phase gazeuse de nitrobenzène et purification consécutive de l'aniline, dans lequel
un produit de réaction brut, gazeux (11/21/31/41) est obtenu, qui est condensé avec fractionnement, de manière à obtenir au moins de produits de procédé liquides, au moins un condensat partiel (PK) (12/22/32/42) et un condensat total (TK) (111/211/311/411),
la condensation conduisant à la formation de PK (12/22/32/42) étant réalisée à une température plus élevée que celle de la condensation conduisant à la formation de TK (111/211/311/411), **caractérisé en ce que**
un flux de produits provenant de PK et un flux de produits provenant de TK sont guidés dans une colonne de distillation (T2) comprenant au moins deux sections, à savoir au moins une partie d'appauvrissement et au moins une partie de rectification,
le flux de produits provenant de PK est guidé dans la partie inférieure de la colonne de distillation entre la partie d'appauvrissement la plus inférieure et la section consécutive et le flux de produits provenant de TK est guidé en tête de la colonne de distillation au-dessus de la partie de rectification la plus supérieure, et
l'aniline distillée (122/222/322/422) est prélevée dans un flux latéral entre la partie d'appauvrissement la plus inférieure et la partie de rectification la plus supérieure de la colonne de distillation.

2. Procédé selon la revendication 1,
la condensation conduisant à la formation de PK (12/22/32/42) étant réalisée à des températures de 60°C à 160°C et
la condensation conduisant à la formation de TK (111/211/311/411) étant réalisée à des températures de 25°C à 90°C.

3. Procédé selon la revendication 1 ou 2,
le flux de produits provenant de PK étant un condensat partiel obtenu dans la condensation avec fractionnement (42) et
le flux de produits provenant de TK étant une phase organique (414) qui est obtenue par séparation de phases de TK (411) ou d'un produit de procédé contenant TK (4111/41111) en une phase aqueuse et une phase organique.

4. Procédé selon la revendication 1 ou 2,
le flux de produits provenant de PK étant une phase organique (117/217/317) qui est obtenue après mélange de PK (12/22/32) ou d'un produit de procédé contenant PK (161/261/361) avec une solution aqueuse basique (17/27/37) et séparation consécutive de phases en une phase aqueuse et une phase organique et
le flux de produits provenant de TK étant une phase organique (116/214/316) qui est obtenue après mélange de TK (111/211/311) ou d'un produit de procédé contenant TK (2111/3111) avec une solution aqueuse basique (112/212/312) et séparation consécutive de phases en une phase aqueuse et une phase organique.

5. Procédé selon la revendication 4,
PK (12/22/32) ou le produit de procédé contenant PK (161/261/361) étant mélangé avant, pendant ou après le mélange avec la solution aqueuse basique (17/27/37) dans un procédé à un ou plusieurs étages avec de l'eau ou un flux A contenant au moins 50% en masse d'eau, par rapport à la masse totale de ce flux A.

6. Procédé selon la revendication 5,
le flux A contenant au moins 50% en masse d'eau étant une phase aqueuse (113/213/313) qui est obtenue après mélange de TK (111/211/311) ou d'un produit de procédé contenant TK (2111/3111) avec une solution aqueuse basique (112/212/312) et séparation consécutive de phases en une phase aqueuse et une phase organique.

7. Procédé selon l'une quelconque des revendications 4 à 6,
la phase organique (114/314) obtenue après le mélange de TK (111/311) ou d'un produit de procédé contenant TK (3111) avec une solution aqueuse basique (112/312) et séparation consécutive des phases étant lavée dans un procédé à un ou plusieurs étages avec de l'eau ou un flux B contenant au moins 50% en masse d'eau, par rapport à la masse totale de ce flux B.

8. Procédé selon la revendication 7,
le flux B contenant au moins 50% en masse d'eau étant un flux de condensat liquide (120) de la phase gazeuse (118) soutirée via la tête de la colonne de distillation (T2) ou un produit de procédé (320) contenant un flux de condensat liquide de la phase gazeuse (318) soutirée via la tête de la colonne de distillation (T2).

9. Procédé selon l'une quelconque des revendications 4 à 6,
TK (211) ou le produit de procédé contenant TK (2111) étant mélangé avant, pendant ou après le mélange avec la solution aqueuse basique (212) dans un procédé à un ou plusieurs étages avec de l'eau ou un flux B contenant au moins 50% en masse d'eau, par rapport à la masse totale de ce flux B.

10. Procédé selon la revendication 9,
le flux B contenant au moins 50% en masse d'eau étant un produit de procédé (220) contenant le flux de condensat liquide de la phase gazeuse (218) soutirée via la tête de la colonne de distillation (T2).

11. Procédé selon la revendication 8,
le produit de procédé (320) contenant le flux de condensat liquide de la phase gazeuse soutirée via la tête de la colonne de distillation (T2) étant obtenu
par addition d'un azéotrope d'aniline-eau (328),
obtenu en faisant passer une phase aqueuse (326), obtenue par séparation des phases après le mélange de PK (32) ou d'un produit de procédé contenant PK (313) avec une solution aqueuse basique (37), dans un rectificateur d'eau (T3),
au condensat total (319) de la phase gazeuse (318) soutirée via la tête de la colonne de distillation (T2).

12. Procédé selon la revendication 10,
le produit de procédé (220) contenant le flux de condensat liquide de la phase gazeuse soutirée via la tête de la colonne de distillation (T2) étant obtenu
par addition d'un azéotrope d'aniline-eau (228),
obtenu en faisant passer une phase aqueuse (226), obtenue par séparation des phases après le mélange de PK (22) d'un produit de procédé contenant PK (213) avec une solution aqueuse basique (27), dans un rectificateur d'eau (T3),
au condensat total (219) de la phase gazeuse (218) soutirée via la tête de la colonne de distillation (T2).

13. Procédé selon la revendication 3, l'aniline (422) prélevée de la colonne de distillation (T2) dans un flux latéral étant mélangée avec une solution aqueuse basique (432) et une aniline appauvrie en impuretés acides (434) étant obtenue par une séparation des phases.

14. Procédé selon l'une quelconque des revendications 4 à 11, une solution d'un hydroxyde de métal alcalin ou d'un hydroxyde de métal alcalinoterreux étant utilisée comme solution aqueuse basique (17/27/37 - 112/212/312 - 432).
